(19) **Europäisches Patentamt / European Patent Office / Office européen des brevets**

(11) **EP 3 636 244 A1**

(12) # EUROPEAN PATENT APPLICATION
published in accordance with Art. 153(4) EPC

(43) Date of publication:
**15.04.2020 Bulletin 2020/16**

(21) Application number: **18801773.5**

(22) Date of filing: **16.05.2018**

(51) Int Cl.:
*A61K 8/06* (2006.01)    *A61K 8/37* (2006.01)
*A61K 8/891* (2006.01)    *A61Q 5/00* (2006.01)
*A61Q 19/00* (2006.01)

(86) International application number:
**PCT/JP2018/018874**

(87) International publication number:
**WO 2018/212222 (22.11.2018 Gazette 2018/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.05.2017 JP 2017100141**

(71) Applicant: **Shiseido Co., Ltd.**
**Tokyo 104-0061 (JP)**

(72) Inventors:
• **SANO, Shusuke**
**Yokohama-shi**
**Kanagawa 224-8558 (JP)**

• **MATSUSHITA, Yuji**
**Yokohama-shi**
**Kanagawa 224-8558 (JP)**
• **SATO, Eiko**
**Yokohama-shi**
**Kanagawa 224-8558 (JP)**
• **TOMINAGA, Naoki**
**Yokohama-shi**
**Kanagawa 224-8558 (JP)**
• **HASEGAWA, Yoriko**
**Yokohama-shi**
**Kanagawa 224-8558 (JP)**

(74) Representative: **Ter Meer Steinmeister & Partner Patentanwälte mbB**
**Nymphenburger Straße 4**
**80335 München (DE)**

(54) **OIL-IN-WATER TYPE EMULSION COSMETIC**

(57)    A purpose of the present invention is to provide an oil-in-water emulsion cosmetic that is able to stably maintain a good dispersion state of large oil-based particles exceeding 50 $\mu$m, or that can be put in a good dispersion state simply by being lightly shaken, without necessarily adding a water-soluble thickener, such as a carboxyvinyl polymer, to the water phase.

The present invention relates to an oil-in-water emulsion cosmetic in which oil-based particles containing a solid oil and a liquid oil are dispersed in a water phase, the oil-in-water emulsion cosmetic being characterized in that the oil-based particles have an average particle size of 50 $\mu$m to 10 mm, and the difference between the specific gravity of the oil phase constituting the oil-based particles and the specific gravity of the water phase [(specific gravity of oil phase) - (specific gravity of water phase)] is within a range from -0.010 to + 0.100.

EP 3 636 244 A1

**Description**

TECHNICAL FIELD

The present invention relates to an oil-in-water emulsion cosmetic. More specifically, the present invention relates to an oil-in-water emulsion cosmetic having oil-based particles that are large enough to be visible, wherein the oil-based particles are well-dispersed in a water phase.

BACKGROUND ART

Oil-in-water emulsion cosmetics in which large oil-based particles having an average particle size of 50 μm to 10 mm are dispersed in a water phase are known. Such oil-in-water emulsion cosmetics containing large oil-based particles are not only visually innovative and appealing because large, visible oil-based particles are dispersed therein, but they also provide an unprecedented feeling in use in which they have a watery and fresh texture when applied to the skin, which becomes a moist texture over time. Additionally, if decomposable oil-soluble components are held in the large oil-based particles, they also have the effect of being able to suppress the decomposition of said oil-soluble components.

For example, Patent Document 1 describes a capsule-containing composition in which oil-based components including an amphiphilic substance that is solid at standard temperature, such as behenyl alcohol, are dispersed in an aqueous solvent in the form of oil-based capsules having an average particle size of 100 μm or more. However, in order to disperse oil-based particles (capsules) that are 100 μm or larger in a water phase, it is necessary to thicken the water phase, and in the examples in Patent Document 1, a carboxyvinyl polymer is added as a water-soluble thickener.

Patent Document 2 describes an external skin preparation in which a polyoxyethylene associative thickener is further added for the purpose of improving on the reduced skin compatibility and the like caused by the carboxyvinyl polymer that was added in Patent Document 1.

RELATED ART

PATENT DOCUMENTS

Patent Document 1: JP 4798899 B
Patent Document 2: JP 2012-67024 A

SUMMARY OF THE INVENTION

PROBLEM TO BE SOLVED BY THE INVENTION

In view of the above-mentioned conventional art, an objective of the present invention is to provide an oil-in-water emulsion cosmetic that is able to stably maintain a good dispersion state of large oil-based particles exceeding 50 μm, or that can be put in a good dispersion state simply by being lightly shaken, without necessarily adding a water-soluble thickener, such as a carboxyvinyl polymer, to the water phase.

MEANS FOR SOLVING THE PROBLEM

The present inventors performed diligent investigations, as a result of which they discovered, for the first time, that the aforementioned problem can be solved by appropriately adjusting the specific gravity of the oil phase constituting the oil-based particles (capsules) and the specific gravity of the water phase in which they are dispersed, thereby completing the present invention.

In other words, the present invention provides an oil-in-water emulsion cosmetic in which oil-based particles containing a solid oil and a liquid oil are dispersed in a water phase, the oil-in-water emulsion cosmetic being characterized in that the oil-based particles have an average particle size of 50 μm to 10 mm, and the difference between the specific gravity of the oil phase constituting the oil-based particles and the specific gravity of the water phase [(specific gravity of oil phase) - (specific gravity of water phase)] is within the range from -0.010 to + 0.100.

EFFECTS OF THE INVENTION

According to the present invention, a stable dispersion state is maintained, or a good dispersion state can be achieved simply by light shaking, even in the absence of a water-soluble thickener, which was conventionally thought to be indispensable for good dispersion of large oil-based particles exceeding 50 μm. Furthermore, by blending a powder into the oil phase, it is possible to improve the uniformity of the shapes and particle sizes of the dispersed oil-based particles. In the present specification, "uniform" means that emulsified particles are dispersed without coalescence or aggregation.

In the cosmetic of the present invention, because there is an appropriate specific gravity difference between the water phase and the oil phase, the oil-based particles will gradually settle without rising above the water phase, even when the cosmetic is left to stand after dispersion.

MODES FOR CARRYING OUT THE INVENTION

The cosmetic according to the present invention is an oil-in-water emulsion cosmetic in which oil-based particles (capsules) having an average particle size of 50 μm to 10 mm and having a certain degree of hardness are dispersed in a water phase. A cosmetic in such a form can be considered to be an "aqueous liquid cosmetic" in which oil-based particles (capsules) are uniformly dispersed. Additionally, when the cosmetic in the aforementioned emulsified (dispersed) form is allowed to stand for a long time, the water phase and the oil phase may separate into upper and lower layers, but the cosmetic can be easily returned to an emulsified (dispersed) state (redispersed) by being lightly shaken.

In the present specification, a cosmetic in a state in which oil-based particles are dispersed will be referred to as an "oil-in-water emulsion cosmetic" (sometimes simply an "emulsion cosmetic"), and when it is necessary to refer to a state in which the water phase and the oil phase are temporarily separated, this will be referred to as a "cosmetic in separated form". Meanwhile, an "(aqueous) liquid cosmetic" can be understood as representing a form including both an oil-in-water emulsion cosmetic and a cosmetic in separated form.

The "oil phase" (also sometimes referred to as the "internal phase" or the "dispersed phase") constituting the oil-based particles in the emulsion cosmetic of the present invention includes a solid oil and a liquid oil, and preferably further contains a powder.

The "solid oil" in the present invention refers to an oil that is solid or semi-solid at ambient temperature (25 °C). Although the solid oil that is used in the present invention is not particularly limited, a solid oil having a melting point of 55 °C or higher is preferably used. Specific examples of the solid oil in the present invention include, but are not limited to, those listed below.

Solid paraffin, microcrystalline wax, ceresin, beeswax, Bareco wax, polyethylene wax, silicon wax, higher alcohols (for example, behenyl alcohol, stearyl alcohol, cetyl alcohol, etc.), batyl alcohol, carnauba wax, cera alba, candelilla wax, jojoba wax, lanolin, shellac wax, spermaceti, Japan wax, higher fatty acids (for example, myristic acid, palmitic acid, stearic acid, behenic acid, 12-hydroxystearic acid, etc.), ester oils (for example, myristyl myristate, etc.), cacao butter, hardened castor oil, hardened oils, hydrogenated palm oil, palm oil, hardened coconut oil, polyethylene, vaseline, various hydrogenated animal or vegetable oils and fats, aliphatic monocarboxylic acid lanolin alcohol esters, and the like.

Among the above, solid oils having a melting point of 50 °C or higher are preferred, and solid oils having a melting point of 65 °C or higher and lower than 85 °C are particularly preferred. Although there are no limits on the preferred solid oils, examples include hydrogenated jojoba oil (melting point 68 °C), glyceryl behenate/eicosadioate (melting point 66 °C), higher alcohols having 16 or more carbon atoms, preferably 18 or more carbon atoms, such as stearyl alcohol (melting point 52 to 62 °C) and behenyl alcohol (melting point 68 °C), microcrystalline wax (melting point 80 °C), ceresin (melting point 68 to 75 °C), polyethylene wax (melting point 80 °C), batyl alcohol (melting point 70 °C), carnauba wax (melting point 83 °C), candelilla wax (melting point 71 °C), hardened castor oil (melting point 84 °C), stearic acid (melting point 58 to 63 °C), behenic acid (melting point 69 to 80 °C) and 12-hydroxystearic acid (melting point 70 °C).

These solid oils may be used as one type alone or as a combination of two or more types.

The blended amount of the solid oil in the cosmetic of the present invention is preferably 5 to 50% by mass, more preferably 10 to 20% by mass relative to the overall mass of the oil phase (excluding the powder). If the blended amount of the solid oil is less than 5% by mass, then there is a tendency for the stability of the cosmetic to become worse, and if it exceeds 50% by mass, then there is a tendency for the oil-based particles to become too hard, thereby making the texture and the skin compatibility worse.

The "liquid oil" in the present invention refers to an oil that is liquid at ambient temperature (25 °C). Specific examples of the liquid oil used in the present invention include, but are not limited to, those listed below..

Oils and fats such as linseed oil, camellia oil, macadamia nut oil, corn oil, olive oil, avocado oil, sasanqua oil, castor oil, safflower oil, apricot kernel oil, cinnamon oil, jojoba oil, grape oil, almond oil, rapeseed oil, sesame oil, sunflower oil, wheat germ oil, rice germ oil, rice bran oil, cottonseed oil, soybean oil, peanut oil, tea seed oil, evening primrose oil, triglycerin, glyceryl trioctanoate, glyceryl triisopalmitate, coconut oil, palm oil and palm kernel oil.

Ester oils including succinic acid esters such as diethoxyethyl succinate, octanoic acid esters such as cetyl octanoate, isooctanoic acid esters such as glyceryl tri-2-ethylhexanoate and pentaerythrityl tetraethylhexanoate, lauric acid esters such as hexyl laurate, myristic acid esters such as isopropyl myristate and octyldodecyl myristate, palmitic acid esters such as octyl palmitate, stearic acid esters such as isocetyl stearate, isostearic acid esters such as isopropyl isostearate, isopalmitic acid esters such as octyl isopalmitate, oleic acid esters such as isodecyl oleate, adipic acid diesters such as diisopropyl adipate, sebacic acid diesters such as diethyl sebacate, and diisostearyl malates such as diisostearyl malate. Diethoxyethyl succinate is particularly preferred.

Hydrocarbon oils such as liquid paraffin, ozokerite, squalane, squalene, pristane, paraffin, isoparaffin and vaseline.

Silicone oils including phenyl group-containing silicone oils such as dimethylpolysiloxane (dimethicone) and methyl phenyl polysiloxane (phenyl methicone), chain silicone oils such as methyl hydrogen polysiloxane, cyclic silicone oils such as decamethylcyclopentasiloxane and dodecamethylcyclohexasiloxane, and modified silicones such as amino-modified silicone oils, polyether-modified silicone oils, carboxy-modified silicone oils, alkyl-modified silicone oils, ammonium salt-modified silicone oils and fluorine-modified silicone oils. In particular, phenyl group-containing silicone oils and fluorine-modified silicone oils are preferred.

These liquid oils may be used as one type alone or may be used as a combination of two or more types.

The blended amount of the liquid oil is preferably 50 to 95% by mass, more preferably 60 to 90% by mass relative to the overall mass of the oil phase (excluding the powder). If the blended amount of the liquid oil in the oil phase is less than 50% by mass, there is a tendency for the oil-based particles to become too hard, thereby making the texture and the skin compatibility worse, and if it exceeds 95% by mass, there is a tendency for the stability of the cosmetic to become worse.

The water phase (also sometimes referred to as the "external phase" or the "continuous phase") in the emulsion cosmetic of the present invention contains water and/or an aqueous medium as the main component, and serves as the dispersion medium for the oil-based particles.

The blended amount of the water constituting the water phase in the cosmetic of the present invention is not particularly limited, but is normally 50 to 99% by mass, preferably 60 to 98% by mass, more preferably 70 to 95% by mass relative to the overall mass of the water phase.

In the cosmetic (the liquid cosmetic including oil-in-water emulsion cosmetics and cosmetics in separated form) of the present invention, the difference between the specific gravity of the oil phase constituting the oil-based particles and the specific gravity of the water phase [(specific gravity of oil phase) - (specific gravity of water phase)] is within the range from -0.010 to + 0.100, preferably -0.005 to +0.050, and more preferably -0.002 to +0.050.

In this case, "specific gravity of the oil phase" and "specific gravity of the water phase" refer to the specific gravities when all of the components contained in the respective phases are included. In other words, the "specific gravity of the oil phase" is the specific gravity of the entire "oil phase" including optional components such as a powder in addition to the aforementioned solid oil and liquid oil, and the "specific gravity of the water phase" is the specific gravity of the entire "water phase" including optional components such as a humectant in addition to the water and/or the aqueous medium.

In the cosmetic of the present invention, by setting the specific gravities of the oil phase and the water phase to be within the aforementioned ranges, in an oil-in-water emulsion cosmetic, the dispersion state of the oil-based particles can be stably maintained even without thickening the water phase, and in a cosmetic in separated form, a dispersion of the oil-soluble particles can be obtained simply by lightly shaking the cosmetic.

The cosmetic of the present invention preferably has a powder blended into the oil phase. The uniformity of the particle sizes and the shapes of the oil-based particles formed by blending a powder into the oil phase is improved. The "powder" in the present invention is not particularly limited as long as it can be blended into an external skin preparation such as a cosmetic.

Examples of powders include inorganic powders such as talc, mica, kaolin, mica, silk mica (sericite), white mica, gold mica, synthetic mica, red mica, black mica, lithia mica, vermiculite, magnesium carbonate, calcium carbonate, aluminum silicate, barium silicate, calcium silicate, magnesium silicate, strontium silicate, tungstic acid metal salts, magnesium, spherical silica, zeolite, barium sulfate, calcined calcium sulfate (calcined plaster), calcium phosphate, fluorapatite, hydroxyapatite, ceramic powder, metal soap (such as zinc myristate, calcium palmitate or aluminum stearate) and boron nitride; organic spherical powders such as spherical polyamide resin powder (spherical nylon powder), spherical polyethylene, spherical crosslinked poly methyl (meth)acrylate resin powder, spherical polyester, spherical crosslinked polystyrene resin powder, spherical styrene-acrylic acid copolymer resin powder, spherical benzoguanamine resin powder, spherical polytetrafluoroethylene powder and spherical cellulose; inorganic white pigments such as titanium dioxide and zinc oxide; inorganic red pigments such as iron oxide (red iron oxide) and iron titanate; inorganic brown pigments such as $\gamma$-iron oxide; inorganic yellow pigments such as yellow iron oxide and ocher; inorganic black pigments such as black iron oxide, carbon black and lower titanium oxides; inorganic violet pigments such as mango violet and cobalt violet; inorganic green pigments such as chromium oxide, chromium hydroxide and cobalt titanate; inorganic blue pigments such as ultramarine blue and Prussian blue; pearlescent pigments such as titanium oxide-coated mica, titanium oxide-coated bismuth oxychloride, titanium oxide-coated talc, colored titanium oxide-coated mica, bismuth oxychloride, (Ca/Al) borosilicate and argentine; metal powder pigments such as aluminum powder and copper powder; red, yellow, orange, yellow, green or blue colorants, or colorants (organic pigments) in which these are formed into lakes with zirconium, barium, aluminum or the like; and natural pigments such as chlorophyll and $\beta$-carotene. Among these, talc is particularly preferred.

The powder in the present invention may or may not be surface-treated, and the shape thereof is not particularly limited. The average particle size of the powder is not particularly limited, but normally, a powder of approximately 1 to 100 $\mu$m is preferably used.

The blended amount of the powder is preferably 0.12 to 30% by mass, more preferably 0.15 to 15% by mass, and even more preferably 0.3 to 9% by mass relative to the overall mass of the oil phase (excluding the powder). If the blended amount of the powder in the oil phase is less than 0.12% by mass, then there is a tendency for the dispersibility of the oil-based particles to be reduced and for aggregation to easily occur. Conversely, if the blended amount exceeds 30% by mass, then there is a tendency for the the cosmetic to be difficult to handle during preparation, and for the particle sizes and variability to become greater.

The cosmetic of the present invention may contain, in addition to the above-mentioned components, other components that can be blended into external skin preparations, such as cosmetics, within a range not inhibiting the effects of the present invention.

Although the other components that can be blended into the cosmetic of the present invention are not limited, examples include water-soluble thickeners, oil-soluble thickeners, humectants, water-soluble medicinal agents (such as, for example, albutin, ascorbic acid glucoside, tranexamic acid and 4-methoxysalicylic acid salts), oil-soluble medicinal agents (such as, for example, oil-soluble vitamins and oil-soluble vegetable extracts), ultraviolet absorption agents, chelating agents such as sodium edetate, pH adjusters such as citric acid/sodium citrate, preservatives such as paraben and phenoxyethanol, colorants, dyes, fragrances, surfactants, and the like.

In the cosmetic of the present invention, the dispersion state of the oil-based particles can be stably maintained even without blending in a water-soluble thickener, but the stability can be further improved by blending in a water-soluble thickener.

Although the water-soluble thickeners that can be blended in the present invention are not particularly limited, examples include vegetable polymers such as gum arabic, tragacanth gum, galactan, carob gum, guar gum, karaya gum, carrageenan, pectin, agar, quinceseed (marmelo) and algecolloid (brown algae extract); microbial polymers such as dextran, succinoglucan and pullulan; animal polymers such as collagen, casein, albumin and gelatin; cellulose polymers such as methylcellulose, nitrocellulose, ethylcellulose, methylhydroxypropylcellulose, hydroxyethylcellulose, sodium cellulose sulfate, hydroxypropylcellulose, sodium carboxymethylcellulose, crystalline cellulose and cellulose powder; alginate polymers such as sodium alginate and alginic acid propylene glycol esters; vinyl polymers such as polyvinyl alcohol, polyvinyl methyl ether, polyvinylpyrrolidone, carboxyvinyl polymers and alkyl-modified carboxyvinyl polymers; acrylic polymers such as polyoxyethylene polymers, polyoxyethylene-polyoxypropylene copolymers, sodium polyacrylate, poly ethyl acrylate and polyacrylamide; and inorganic water-soluble polymers such as polyethyleneimine, cationic polymers, bentonite, aluminum-magnesium silicate, laponite, hectorite and silicic anhydride.

Among water-soluble thickeners, alkyl-modified carboxyvinyl polymers are particularly preferred because they provide the function of suppressing the aggregation and coalescence of oil-based particles based on the surface activity thereof. Alkyl-modified carboxyvinyl polymers are also known as acrylate/alkyl methacrylate copolymers (acrylates/alkyl acrylate ($C_{10-30}$) crosspolymers), and are commercially available, for example, under the trade names Carbopol 1342, Pemulen TR-1 and Pemulen TR-2 (manufactured by BF Goodrich).

The amount of the water-soluble thickener can be appropriately adjusted in accordance with the type of water-soluble thickener, the purpose of use, and the desired texture, and there are no particular limitations thereto. Normally, the amount should be 0.001 to 2% by mass, preferably 0.01 to 1.3% by mass relative to the overall mass of the cosmetic.

The oil-soluble thickener is adjustable to provide a suitable hardness by lowering the solidification power of the solid oil constituting the oil-based particles. In particular, it is preferable for the oil-soluble thickener to be blended when a wax such as candelilla wax or the like is used as the solid oil.

Although the oil-soluble thickeners that can be blended in the present invention are not particularly limited, examples include dextrin fatty acid esters, metal soaps, lipophilic bentonites, amino acid derivatives, sucrose fatty acid esters, benzylidene derivatives of sorbitol, and the like.

Examples of dextrin fatty acid esters include dextrin palmitic acid esters, dextrin oleic acid esters, dextrin stearic acid esters, and the like.

Examples of metal soaps include aluminum stearate, magnesium stearate, zinc myristate and the like having residual hydroxyl groups.

Examples of lipophilic bentonites include dimethylbenzyldodecylammonium montmorillonite, dimethyldioctadecylammonium montmorillonite, and the like.

Examples of amino acid derivatives include N-lauroyl-L-glutamic acid, $\alpha,\gamma$-di-n-butylamine, and the like.

Examples of sucrose fatty acid esters include those in which three or fewer among eight hydroxyl groups are esterified with a higher fatty acid, and the higher fatty acid is stearic acid or palmitic acid.

Examples of benzylidene derivatives of sorbitol include monobenzylidene sorbitol, dibenzylidene sorbitol, and the like.

Among these, dextrin palmitate is particularly preferred in view of the stability and texture. The dextrin fatty acid esters are not particularly limited, but for example, those that are commercially available from Chiba Flour Milling Co., Ltd. under the tradenames "Rheopearl KL", "Rheopearl KE" and the like may be used.

The amount of the oil-soluble thickener can be appropriately adjusted in accordance with the purpose of use and the desired feeling in use, and there are no particular limitations thereon. Normally, the amount should be 0.01 to 5% by mass, preferably 0.02 to 2% by mass relative to the overall mass of the oil phase.

The cosmetic of the present invention can be prepared, for example, in accordance with the method described in Patent Document 1 or 2. Specifically, it can be prepared by mixing the water phase components together, heating the water phase to at least the melting point of the solid oil to be blended, adding the oil phase components (including the solid oil and the liquid oil, and optionally the powder), which have been heated to the same temperature and mixed, to the water phase components while stirring (the stirring may be implemented by using a propeller or paddle mixer having a rotation speed of 10 to 1500 rpm, preferably 20 to 300 rpm; a homomixer is preferably not used because the particles may then become too fine). Next, the mixture can be cooled while stirring to prepare the cosmetic.

The cosmetic of the present invention that is produced in this manner has oil-based particles having an average particle size of 50 $\mu$m to 10 mm dispersed in a water phase. The oil-based particles in the cosmetic of the present invention can be considered to have a capsule structure in which an inner layer is substantially composed of the liquid oil (optionally including the powder), and the periphery thereof is coated with a solid oil (outer layer). Additionally, the solid oil forming the outer layer crystallizes, thereby becoming an appropriate hardness and providing a unique texture in use.

In the cosmetic of the present invention, oil-based particles having the above-mentioned structure are formed even without using a surfactant, but when the powder is blended in, the cosmetic is clearly distinguishable from a Pickering emulsion, in which a powder is present at the oil-water interface, in that the powder is present inside (in the inner layer of) the oil-based particles and there is a solid oil at the interface between the water phase and the oil phase.

In the present invention, if a colorant such as a pigment is to be blended as the powder, it is preferable to also use a small amount (for example, 0.005% by mass or less) of a surfactant. The surfactant is preferably lipophilic, with an HLB of 7 or lower, and specific examples include PEG-9 polydimethylsiloxyethyl dimethicone, sorbitan sesquiisostearate, and the like.

The oil-in-water emulsion cosmetic of the present invention is suitable for use as a cosmetic base having a unique appearance and feeling in use. For example, by blending coloring pigments, coloring dyes or the like into the oil phase, it is possible to obtain a visually appealing cosmetic as a liquid cosmetic in which colored oil-based particles that are of a visible size are uniformly dispersed.

Additionally, by making "specific gravity of the oil phase" > "specific gravity of the water phase", the cosmetic in separated form is separated with the water phase on the upper side and the oil phase on the lower side. By using the cosmetic in separated form, there is initially a watery texture due to the water phase, but with light shaking, the cosmetic enters an emulsified form in which the oil-based particles are easily dispersed, thereby also allowing a unique feeling in use providing a moist texture to be obtained.

As specific product forms, the cosmetic may be used, for example, as a facial, body-care or hair-care cosmetic such as a lotion, a milky lotion, a cream or a pack.

EXAMPLES

While the present invention will be explained in further detail by providing examples below, the present invention is not limited by these examples in any way. Where not otherwise noted, the blended amounts are indicated in % by mass relative to the overall mass of the cosmetic in which those components are blended.

A water phase was prepared with the formulation indicated in Table 1 below. The specific gravity thereof was "1.010". The specific gravity of the water phase was measured by a U-tube vibration system (usinng an Anton-Paar DMA 4100 M).

The specific gravity of the oil phase was measured in the following way. A cylindrical metal can (weight Wc) was filled with water at 20 °C, then let stand for one hour in a 20 °C constant-temperature tank. The weight (Ww) was measured at that time. Next, the oil phase was melted, and approximately 80% of the whole was poured into the metal can and solidified. Since the solidified oil contracted, the remaining 20% was poured into the resulting space and solidified. At this time, the oil was poured in such a manner that the liquid surface bulged upward, then solidified in the 20 °C constant-temperature tank. After complete solidification, the part bulging above the surface of the metal can was cut, and the overall weight (Ws) was measured. The specific gravity was computed, from the obtained weights, by using the following equation:

$$\text{Specific gravity of oil phase} = (Ws - Wc)/(Ww - Wc)$$

[Table 1]

| Ion-exchanged water | Balance |
|---|---|
| EDTA-2Na 2H$_2$O | 0.02 |
| Citric acid (edible) | 0.02 |
| Sodium citrate | 0.08 |
| 1,3-Butylene glycol | 7 |
| Dipropylene glycol | 5 |
| Phenoxyethanol | 0.5 |
| General industrial alcohol, 95% | 2 |
| Dynamite glycerin | 2 |
| Total | 100 |
| Specific gravity (water phase) | 1.010 |

Next, the water phase described in Table 1 above was combined with oil phases having the formulations listed in Table 2 below to prepare oil-in-water emulsion cosmetics. The cosmetic of each example was evaluated regarding (1) the formability (dispersibility) of the oil-based particles at the time of production, and (2) the re-formability of the oil-based particles due to shaking after having been put in a separated form by being allowed to stand for a predetermined period of time, using the methods and criteria indicated below.

(1) Oil-based particle formability (dispersibility) at time of production

The state of the oil-based particles contained in a prepared emulsion cosmetic was observed by eye and under a microscope to evaluate the particle formability and the dispersion state.

Evaluation criteria

A: Particles were well-formed, the average particle size of the formed particles was in the range from 50 $\mu$m to 10 mm, and the particles were uniformly (without coalescence or aggregation) dispersed in a water phase
B: Particles were well-formed, the average particle size of the formed particles was in the range from 50 $\mu$m to 10 mm, but a small amount (less than 3%) of the particles rose to the surface of the water phase
C: Particles were well-formed, the average particle size of the formed particles was in the range from 50 $\mu$m to 10 mm, but some (at least 3% and less than 10%) of the particles rose to the surface of the water phase
D: Particles were not formed, and instead, lumps were formed, or the liquid clouded, or even if particles were formed, at least half of the particles rose to the surface of the water phase

(2) Particle re-formability (re-dispersibility) from separated form

After a prepared emulsion cosmetic was allowed to stand for one hour at ambient temperature and thus put in a separated form, the oil-based particle formability (re-dispersibility) and the state of the water phase, when the cosmetic was agitated for one hour at a speed of 50 rpm using a shaking machine (MILD MIXER), were evaluated in accordance with the criteria in (1) above.

[Table 2]

| | Ex. 1 | Ex. 2 | Ex. 3 | Ex. 4 | Ex. 5 | Ex. 6 | Ex. 7 |
|---|---|---|---|---|---|---|---|
| Water phase (Table 1) | bal | bal | bal | bal | bal | bal | bal |
| Behenyl alcohol | 0.08 | - | 0.04 | 0.3 | 0.5 | 0.3 | - |
| Stearyl alcohol | - | 0.16 | - | - | - | | - |
| Batyl alcohol | 0.08 | 0.08 | 0.04 | 0.3 | 0.5 | 0.1 | - |
| Candelilla wax | - | - | - | - | - | | 0.87 |
| Dextrin palmitate | - | - | - | - | - | - | 0.05 |
| Trimethyl pentaphenyl trisiloxane | 0.4 | 0.4 | 0.4 | 0.4 | 0.4 | 1.7 | 2.8 |
| Methyl phenyl polysiloxane (specific gravity 1.07)* | 0.4 | 0.4 | 0.4 | 0.4 | 0.4 | - | - |
| Diethoxyethyl succinate | - | - | - | - | | 0.4 | - |
| Pentaerythritol tetra-2-ethylhexanoate | 0.04 | 0.04 | 0.04 | 0.04 | 0.04 | - | 0.25 |
| Acrylic acid-alkyl methacrylate copolymer | - | - | - | - | - | - | 0.01 |
| Total | 100 | 100 | 100 | 100 | 100 | 100 | 100 |
| Specific gravity (oil phase) | 1.049 | 1.028 | 1.056 | 1.010 | 0.981 | 1.038 | 1.046 |
| Specific gravity difference (oil phase - water phase) | 0.039 | 0.018 | 0.046 | 0.000 | -0.029 | 0.028 | 0.036 |
| Particle formability (dispersibility) at time of production | A | A | A | A | D(rose) | A | A |
| Particle re-formability (re-dispersibility) from separated form | A | B | A | A | - | A | A |

| | Ex. 8 | Ex. 9 | Ex. 10 | Ex. 11 | Ex. 12 | Ex. 13 |
|---|---|---|---|---|---|---|
| Water phase (Table 1) | bal | bal | bal | bal | bal | bal |
| Behenyl alcohol | 0.08 | 0.08 | 0.08 | 0.08 | 0.08 | - |
| Stearyl alcohol | 0.08 | 0.08 | 0.08 | 0.08 | 0.08 | - |
| Batyl alcohol | - | - | - | - | - | - |
| Candelilla wax | - | - | - | - | - | - |
| Microcrystalline wax/paraffin wax mixture | - | - | - | - | - | 0.6 |
| Dextrin palmitate | - | - | - | - | - | - |
| Trimethyl pentaphenyl trisiloxane | 0.4 | 0.4 | 0.4 | 0.4 | 0.4 | 0.4 |
| Methyl phenyl polysiloxane (specific gravity 1.07)* | 0.4 | 0.3 | 0.2 | 0.1 | - | - |
| Pentaerythritol tetra-2-ethylhexanoate | 0.04 | 0.04 | 0.04 | 0.04 | 0.04 | 1.85 |
| Methyl phenyl polysiloxane (specific gravity 0.995)* | - | 0.1 | 0.2 | 0.3 | 0.4 | - |
| Dimethicone | - | - | - | - | - | 1.6 |
| Acrylic acid-alkyl methacrylate copolymer | - | - | - | - | - | 0.01 |
| Talc (hydrophobic, particle size 5 μm) | - | - | - | - | - | 1 |
| Total | 100 | 100 | 100 | 100 | 100 | 100 |
| Specific gravity (oil phase) | 1.043 | 1.039 | 1.028 | 1.021 | 1.012 | 1.066 |

(continued)

|  | Ex. 8 | Ex. 9 | Ex. 10 | Ex. 11 | Ex. 12 | Ex. 13 |
|---|---|---|---|---|---|---|
| Specific gravity difference (oil phase - water phase) | 0.033 | 0.029 | 0.018 | 0.011 | 0.002 | 0.056 |
| Particle formability (dispersibility) at time of production | A | A | A | A | A | A |
| Particle re-formability (re-dispersibility) from separated form | A | A | A | A | A | A |
| * Specific gravity value at 25 °C | | | | | | |

As shown in Table 2, when the specific gravity difference between the oil phase and the water phase (oil phase - water phase) was within the range from -0.010 to 0.100, uniform dispersion (without coalescence or aggregation) and re-dispersion were possible. However, in Example 5 in which the specific gravity difference (oil phase - water phase) was -0.029, at least half of the particles rose to the surface of the water phase.

Water phases with the formulations shown in Table 3 below and oil phases with the formulations shown in Table 4 were separately prepared, and oil-in-water emulsion cosmetics were prepared using 99% by mass of the respective water phases and 1% by mass of the respective oil phases. The results of evaluation, in accordance with (1) above, of the particle formability (dispersibility) of each cosmetic at the time of production are shown in Table 5.

[Table 3]

|  | Water phase 01 | Water phase 02 | Water phase 03 | Water phase 04 | Water phase 05 | Water phase 06 | Water phase 07 | Water phase 08 | Water phase 09 | Water phase 10 | Water phase 11 | Water phase 12 |
|---|---|---|---|---|---|---|---|---|---|---|---|---|
| Ion-exchanged water | bal | bal | bal | bal | bal | bal | bal | bal | bal | bal | bal | bal |
| EDTA-2Na 2$H_2$O | 0.02 | 0.02 | 0.02 | 0.02 | 0.02 | 0.02 | 0.02 | 0.02 | 0.02 | 0.02 | 0.02 | 0.02 |
| Citric acid (edible) | 0.02 | 0.02 | 0.02 | 0.02 | 0.02 | 0.02 | 0.02 | 0.02 | 0.02 | 0.02 | 0.02 | 0.02 |
| Sodium citrate | 0.08 | 0.08 | 0.08 | 0.08 | 0.08 | 0.08 | 0.08 | 0.08 | 0.08 | 0.08 | 0.08 | 0.08 |
| 1,3-butylene glycol | 7 | 7 | 7 | 7 | 7 | 7 | 7 | 7 | 7 | 7 | 7 | 4 |
| Dipropylene glycol | 5 | 5 | 5 | 5 | 5 | 5 | 5 | 5 | 5 | 5 | 5 | 5 |
| Phenoxyethanol | 0.5 | 0.5 | 0.5 | 0.5 | 0.5 | 0.5 | 0.5 | 0.5 | 0.5 | 0.5 | 0.5 | 0.5 |
| Ethanol | 11 | 6 | 2 | 2 | 2 | 2 | 2 | 2 | 2 | 2 | 2 | - |
| Dynamite glycerin | - | - | 0.7 | 1.9 | 3.1 | 4.4 | 5.6 | 7 | 8.2 | 9.5 | 11 | 2 |
| Polyethylene glycol 100 | - | - | - | - | - | - | - | - | - | - | - | 1 |
| Total | 100 | 100 | 100 | 100 | 100 | 100 | 100 | 100 | 100 | 100 | 100 | 100 |
| Specific gravity (water phase) | 0.993 | 1.000 | 1.007 | 1.010 | 1.013 | 1.017 | 1.020 | 1.023 | 1.026 | 1.030 | 1.034 | 1.011 |

EP 3 636 244 A1

[Table 4]

|  | Oil phase 01 | Oil phase 02 | Oil phase 03 | Oil phase 04 | Oil phase 05 |
|---|---|---|---|---|---|
| Behenyl alcohol | 8 | 8 | 8 | 8 | 8 |
| Batyl alcohol | 8 | 8 | 8 | 8 | 8 |
| Trimethylpentaphenyl trisiloxane | 40 | 40 | 40 | 40 | 40 |
| Methyl phenyl polysiloxane (specific gravity 1.07)* | 40 | 30 | 20 | 10 | - |
| Pentaerythritol tetra-2-ethylhexanoate | 4 | 4 | 4 | 4 | 4 |
| Methyl phenyl polysiloxane (specific gravity 0.995)* | - | 10 | 20 | 30 | 40 |
| Total | 100 | 100 | 100 | 100 | 100 |
| Specific gravity (oil phase) | 1.043 | 1.039 | 1.028 | 1.021 | 1.012 |
| * Specific gravity value at 25 °C | | | | | |

[Table 5]

|  |  | Oil phase 01 | Oil phase 02 | Oil phase 03 | Oil phase 04 | Oil phase 05 |
|---|---|---|---|---|---|---|
| Water phase 01 | Specific gravity difference (oil-water) | 0.050 | 0.046 | 0.035 | 0.028 | 0.019 |
| | Dispersibility | A | A | A | A | A |
| Water phase 02 | Specific gravity difference (oil-water) | 0.043 | 0.039 | 0.028 | 0.021 | 0.012 |
| | Dispersibility | A | A | A | A | A |
| Water phase 03 | Specific gravity difference (oil-water) | 0.036 | 0.032 | 0.021 | 0.014 | 0.005 |
| | Dispersibility | A | A | A | A | A |
| Water phase 04 | Specific gravity difference (oil-water) | 0.033 | 0.029 | 0.018 | 0.011 | 0.002 |
| | Dispersibility | A | A | A | A | A |
| Water phase 05 | Specific gravity difference (oil-water) | 0.030 | 0.026 | 0.015 | 0.009 | -0.001 |
| | Dispersibility | A | A | A | A | A |
| Water phase 06 | Specific gravity difference (oil-water) | 0.026 | 0.022 | 0.011 | 0.004 | -0.005 |
| | Dispersibility | A | A | A | A | B |
| Water phase 07 | Specific gravity difference (oil-water) | 0.023 | 0.019 | 0.008 | 0.001 | -0.008 |
| | Dispersibility | A | A | A | A | C |
| Water phase 08 | Specific gravity difference (oil-water) | 0.020 | 0.016 | 0.005 | -0.002 | -0.011 |
| | Dispersibility | A | A | A | A | C |

(continued)

| | | Oil phase 01 | Oil phase 02 | Oil phase 03 | Oil phase 04 | Oil phase 05 |
|---|---|---|---|---|---|---|
| Water phase 09 | Specific gravity difference (oil-water) | 0.017 | 0.013 | 0.002 | -0.005 | -0.014 |
| | Dispersibility | A | A | A | B | D(rose) |
| Water phase 10 | Specific gravity difference (oil-water) | 0.013 | 0.009 | -0.002 | -0.009 | -0.018 |
| | Dispersibility | A | A | A | C | D(rose) |
| Water phase 11 | Specific gravity difference (oil-water) | 0.009 | 0.005 | -0.006 | -0.013 | -0.022 |
| | Dispersibility | A | A | A | D(rose) | D(rose) |
| Water phase 12 | Specific gravity difference (oil-water) | 0.032 | 0.028 | 0.017 | 0.010 | 0.002 |
| | Dispersibility | A | A | A | A | A |

As shown in Table 5, by setting the difference between the specific gravity of the water phase and the specific gravity of the oil phase to be within the range in the present invention, the oil-based particles were able to be uniformly dispersed in the water phase, but a uniform dispersion state could not be maintained when the specific gravity difference was outside the range in the present invention.

Other formulation examples of the cosmetic according to the present invention are shown in Table 6 below. These cosmetics also had good dispersibility/re-dispersibility.

[Table 6]

| | Formulation example 1 | Formulation example 2 | Formulation example 3 | Formulation example 4 |
|---|---|---|---|---|
| Ion-exchanged water | bal | bal | bal | bal |
| EDTA-2Na 2$H_2O$ | 0.02 | 0.02 | 0.02 | 0.02 |
| Citric acid (edible) | 0.02 | 0.02 | 0.02 | 0.02 |
| Sodium citrate | 0.08 | 0.08 | 0.08 | 0.08 |
| 1,3-Butylene glycol | 7 | 7 | 7 | 7 |
| Dipropylene glycol | 5 | 5 | 5 | 5 |
| Phenoxyethanol | 0.5 | 0.5 | 0.5 | 0.5 |
| General industrial alcohol, 95% | 2 | 2 | 2 | 2 |
| Dynamite glycerin | 2 | 2 | 2 | 2 |
| Tranexamic acid | 1 | - | - | - |
| Potassium 4-methoxysalicylate | 1 | - | - | - |
| L-Ascorbic acid 2-glucoside | - | 2 | - | - |
| Potassium hydroxide | - | 042 | - | - |
| Magnesium L-ascorbyl phosphate | - | 0.01 | - | - |
| 3-O-ethyl ascorbic acid | - | - | 0.01 | - |
| Behenyl alcohol | 0.08 | 0.08 | 0.08 | 0.08 |
| Batyl alcohol | 0.08 | 0.08 | 0.16 | 0.16 |

(continued)

| | Formulation example 1 | Formulation example 2 | Formulation example 3 | Formulation example 4 |
|---|---|---|---|---|
| Trimethylpentaphenyl trisiloxane | 0.4 | 0.4 | 0.4 | 0.4 |
| Methyl phenyl polysiloxane (specific gravity 1.07)* | 0.4 | 0.4 | 0.4 | 0.4 |
| Pentaerythritol tetra-2-ethylhexanoate | 0.04 | 0.04 | 0.04 | 0.04 |
| Glyceryl diisostearate | - | 0.01 | - | - |
| Retinol | - | - | - | 0.001 |
| Total | 100 | 100 | 100 | 100 |
| * Specific gravity value at 25 °C | | | | |

## Claims

1. An oil-in-water emulsion cosmetic in which oil-based particles containing a solid oil and a liquid oil are dispersed in a water phase, the oil-in-water emulsion cosmetic being **characterized in that** the oil-based particles have an average particle size of 50 $\mu$m to 10 mm, and the difference between the specific gravity of the oil phase constituting the oil-based particles and the specific gravity of the water phase [(specific gravity of oil phase) - (specific gravity of water phase)] is within a range from -0.010 to + 0.100.

2. The cosmetic as in claim 1, wherein the difference between the specific gravity of the oil phase and the specific gravity of the water phase [(specific gravity of oil phase) - (specific gravity of water phase)] is within a range from -0.005 to + 0.050.

3. The cosmetic as in claim 1 or 2, wherein the solid oil is selected from among solid oils having a melting point that is 50 °C or higher.

4. The cosmetic as in any one of claims 1 to 3, wherein the liquid oil contains a phenyl group-containing silicone oil.

5. The cosmetic as in any one of claims 1 to 3, wherein the liquid oil contains diethoxyethyl succinate.

6. The cosmetic as in any one of claims 1 to 5, wherein the oil phase further contains a powder.

## INTERNATIONAL SEARCH REPORT

| International application No. |
|---|
| PCT/JP2018/018874 |

**A. CLASSIFICATION OF SUBJECT MATTER**
Int.Cl.    A61K8/06(2006.01)i,    A61K8/37(2006.01)i,    A61K8/891(2006.01)i,
           A61Q5/00(2006.01)i, A61Q19/00(2006.01)i

According to International Patent Classification (IPC) or to both national classification and IPC

**B. FIELDS SEARCHED**

Minimum documentation searched (classification system followed by classification symbols)
Int.Cl. A61K8/06, A61K8/37, A61K8/891, A61Q5/00, A61Q19/00

Documentation searched other than minimum documentation to the extent that such documents are included in the fields searched
Published examined utility model applications of Japan       1922-1996
Published unexamined utility model applications of Japan     1971-2018
Registered utility model specifications of Japan             1996-2018
Published registered utility model applications of Japan     1994-2018

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2005-036001 A (SHISEIDO CO., LTD.) 10 February 2005, claims, examples 1, 3-4, 7, prescription examples 4-9, 13-20, paragraphs [0001], [0019] & WO 2006/003733 A1 & CN 1980634 A | 1-6 |
| X | JP 2012-067024 A (SHISEIDO CO., LTD.) 05 April 2012, claims, examples 1-7, prescription examples 1-3, paragraph [0023] (Family: none) | 1-5 |
| A | | 6 |

☒ Further documents are listed in the continuation of Box C.      ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 09 August 2018 (09.08.2018) | 21 August 2018 (21.08.208) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| | International application No. |
|---|---|
| | PCT/JP2018/018874 |

C (Continuation).    DOCUMENTS CONSIDERED TO BE RELEVANT

| Category* | Citation of document, with indication, where appropriate, of the relevant passages | Relevant to claim No. |
|---|---|---|
| A | JP 10-036244 A (ASAHI KASEI INDUSTRY CO., LTD.) 10 February 1998, paragraph [0002] (Family: none) | 1-6 |

Form PCT/ISA/210 (continuation of second sheet) (January 2015) ASAHI KASEI INDUSTRY CO., LTD.

**REFERENCES CITED IN THE DESCRIPTION**

**Patent documents cited in the description**

- JP 4798899 B

- JP 2012067024 A